(19) Europäisches Patentamt / European Patent Office / Office européen des brevets

(11) **EP 3 001 337 B1**

(12) **EUROPEAN PATENT SPECIFICATION**

(45) Date of publication and mention of the grant of the patent:
**15.11.2023  Bulletin 2023/46**

(21) Application number: **15177581.4**

(22) Date of filing: **20.07.2015**

(51) International Patent Classification (IPC):
**G16H 50/20** (2018.01)

(52) Cooperative Patent Classification (CPC):
**G16H 50/20**

(54) **METHOD AND SYSTEM FOR DETERMINING HEALTH CONDITION OF A SUBJECT**

VERFAHREN UND SYSTEM ZUR BESTIMMUNG DES GESUNDHEITSZUSTANDS EINER PERSON

PROCÉDÉ ET SYSTÈME PERMETTANT DE DÉTERMINER UN ÉTAT DE SANTÉ D'UN SUJET

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.09.2014  IN CH46542014**
**18.12.2014  US 201414575449**

(43) Date of publication of application:
**30.03.2016  Bulletin 2016/13**

(73) Proprietor: **Wipro Limited**
**560 035 Karnataka (IN)**

(72) Inventors:
  • **RATH, Satish Prasad**
    **560066 Bangalore (IN)**
  • **SUDDAMALLA, Upendra**
    **515591 Anantapur Dt. (IN)**

(74) Representative: **Finnegan Europe LLP**
**1 London Bridge**
**London SE1 9BG (GB)**

(56) References cited:
US-A1- 2006 247 542    US-A1- 2009 054 743
US-A1- 2010 222 658    US-A1- 2011 199 214

• LI Z. ET AL: "Assessment of cerebral oxygenation during prolonged simulated driving using near infrared spectroscopy: its implications for fatigue development", EUROPEAN JOURNAL OF APPLIED PHYSIOLOGY, SPRINGER, BERLIN, DE, vol. 107, no. 3, 4 July 2009 (2009-07-04), pages 281-287, XP019760329, ISSN: 1439-6327, DOI: 10.1007/S00421-009-1122-6

• NYBO L. ET AL: "Inadequate Cerebral Oxygen Delivery and Central Fatigue during Strenuous Exercise", EXERCISE AND SPORT SCIENCES REVIEWS, vol. 35, no. 3, 1 July 2007 (2007-07-01), pages 110-118, XP055444831, US ISSN: 0091-6331, DOI: 10.1097/jes.0b013e3180a031ec

• JAGANNATH M. ET AL: "Assessment of early onset of driver fatigue using multimodal fatigue measures in a static simulator", APPLIED ERGONOMICS, vol. 45, no. 4, 26 February 2014 (2014-02-26), pages 1140-1147, XP055444851, GB ISSN: 0003-6870, DOI: 10.1016/j.apergo.2014.02.001

• MASLEHATY H. ET AL: "Continuous Measurement of Cerebral Oxygenation with Near-Infrared Spectroscopy after Spontaneous Subarachnoid Hemorrhage", ISRN NEUROLOGY, vol. 2012, 1 January 2012 (2012-01-01), pages 1-7, XP055446627, DOI: 10.5402/2012/907187

• FERNÁNDEZ-SEARA M. A. ET AL: "MR susceptometry for measuring global brain oxygen extraction", MAGNETIC RESONANCE IN MEDICINE., vol. 55, no. 5, 5 April 2006 (2006-04-05), pages 967-973, XP055446435, US ISSN: 0740-3194, DOI: 10.1002/mrm.20892

• BUNDELE M. M. ET AL: "Detection of fatigue of vehicular driver using skin conductance and oximetry pulse : a neural network approach", PROCEEDINGS OF THE 11TH INTERNATIONAL CONFERENCE ON INFORMATION INTEGRATION AND WEB-BASED APPLICATIONS & SERVICES, IIWAS '09, 1 January 2009 (2009-01-01), page 739, XP055446421, New York, New York, USA DOI: 10.1145/1806338.1806478 ISBN: 978-1-60558-660-1

EP 3 001 337 B1

- **MANGOLD R. ET AL: "THE EFFECTS OF SLEEP AND LACK OF SLEEP ON THE CEREBRAL CIRCULATION AND METABOLISM OF NORMAL YOUNG MEN 1", JOURNAL OF CLINICAL INVESTIGATION, vol. 34, no. 7 Pt 1, 1 July 1955 (1955-07-01), pages 1092-1100, XP055445357, US ISSN: 0021-9738, DOI: 10.1172/JCI103158**

Description

## TECHNICAL FIELD

The present subject matter is related, in general to health monitoring and more particularly, but not exclusively to, a method and a system for determining the mental fatigue level of a subject.

## BACKGROUND

Human fatigue is a temporary inability to maintain optimal cognitive performance. The onset of human fatigue during any cognitive activity is gradual, and depends upon an individual's cognitive ability, and also upon other factors, such as sleep deprivation and overall health. Human fatigue has also been shown to adversely affect physical performance.

In recent years, experts have been emphasizing the importance of an objective assessment of human fatigue for preventing deaths caused by accidents and overwork in the automotive and occupational fields. For example, in the mining industry, the driver of a truck who is on extended work durations needs to be attentive to avoid any kind of accident during the transportation to avoid any loss of assets, life and production time. Similarly, a stock trader needs to be attentive to take appropriate decisions quickly to avoid losses. Detection of fatigue is critical in various other fields of work also, such as, mining, aviation, etc. Upon assessing such fatigue, it is important to make the assessment in real time in practical situations, and to notify the user to pay attention to his fatigue, instead of the conventional fatigue assessments made in laboratories. However, the conventional assessments methods were neither non-invasive, nor simple, making the conventional methods difficult to use in practical assessments.

Existing techniques usually measure some physiological parameters related to eye and/or a body part, pressure, Electro Cardiography (ECG), etc. and then try to correlate them to deduce to an overall fatigue of the person. Such measurement techniques cause much inconvenience to the person. Additionally, the existing measurements are directed to assessing the physical fatigue and not for calculating the mental fatigue of the person. The physical fatigue was evaluated relatively easily in reference to some external indices such as muscular weakness, whereas mental fatigue was evaluated mostly by carrying out surveys or the like. Therefore, it has always been difficult to exclude arbitrariness from mental fatigue evaluation.

Also, the present techniques do not detect mental fatigue levels using non-invasive methods. Hence, it becomes difficult to continuously monitor a person's stress levels and ability to maintain optimal performance.

LI Z. et al, "Assessment of cerebral oxygenation during prolonged simulated driving using near infrared spectroscopy: its implications for fatigue development", EUROPEAN JOURNAL OF APPLIED PHYSIOLOGY, SPRINGER, BERLIN, DE, vol. 107, no. 3, 4 July 2009, ISSN: 1439-6327 describes a study of cerebral oxygenation during prolonged simulated driving using near infrared spectroscopy and its implication for fatigue development. NYBO L. et al, "Inadequate Cerebral Oxygen Delivery and Central Fatigue during Strenuous Exercise", EXERCISE AND SPORT SCIENCES REVIEWS, D2 vol. 35, no. 3, 1 July 2007 (2007-07-01), ISSN: 0091-6331 investigates whether, during some exercise conditions, fatigue can be provoked or modulated by inadequate oxygen delivery to the brain and subsequently low cerebral capillary and mitochondrial oxygen tension.

JAGANNATH M. et al, "Assessment of early onset of driver fatigue using multimodal fatigue measures in a static simulator", APPLIED ERGONOMICS, D3 vol. 45, no. 4, 26 February 2014, ISSN: 0003-6870 relates to assessment of early onset of driver fatigue using multimodal fatigue measures in a static simulator. US 2010/0222658 A1 relates to techniques for monitoring vital functions of the human body, including cerebral tissue hemodynamics and, in particular, to an optical method and system for non-invasive and continuous monitoring of cerebral parameters.

US 2006/0247542 A1 relates to a method for evaluating the degree of fatigue of a human body using, as an index, change in waveform of a pulse wave, especially acceleration pulse wave. US 2011/0199214 A1 relates to a method for generating an alert based on a physiological characteristic of a patient, such as heart rate, respiratory rate, cardiac rhythm, blood pressure, oxygen saturation, and blood chemistry, based on comparison of a value of the physiological characteristic to a corresponding alert value.

Therefore, there is a need for a method and a system to monitor mental fatigue level of a subject in a non-invasive manner.

## SUMMARY

One or more shortcomings of the prior art are overcome and additional advantages are provided through the present disclosure. Additional features and advantages are realized through the techniques of the present disclosure. Other embodiments and aspects of the disclosure are described in detail herein and are considered a part of the claimed disclosure.

Disclosed herein is a method for determining health condition of a subject. The method comprises receiving, by a computing unit, a first oxygen saturation level and a second oxygen saturation level from at least two pulse oximetry sensors placed on the subject, wherein the first oxygen saturation level is an oxygen saturation level in incoming blood flow via a carotid artery of the subject and the second oxygen saturation level is an oxygen saturation level in outgoing blood flow via a jugular vein of the subject. Then, the method determines cerebral extraction of oxygen using the first and second oxygen saturation levels by determining a difference between the first and second oxygen saturation levels. Further, the method comprises comparing the cerebral extraction of oxygen with a threshold level and identifying a mental fatigue level of the subject based on outcome of the comparison. The mental fatigue level indicates the health condition of the subject.

In an aspect of the present disclosure, a system for determining health condition of a subject is disclosed. The system comprises a processor and a memory communicatively coupled to the processor. The memory stores processor-executable instructions which on execution, causes the processor to receive a first oxygen saturation level and a second oxygen saturation level from at least two pulse oximetry sensors placed on the subject, wherein the first oxygen saturation level is an oxygen saturation level in incoming blood flow via a carotid artery of the subject and the second oxygen saturation level is an oxygen saturation level in outgoing blood flow via a jugular vein of the subject. Then, the processor determines cerebral extraction of oxygen using the first and second oxygen saturation levels by determining a difference between the first and second oxygen saturation levels. Further, the processor compares the cerebral extraction of oxygen with a threshold level and identifies a mental fatigue level of the subject based on outcome of the comparison. The mental fatigue level indicates the health condition of the subject.

In another aspect of the present disclosure, a non-transitory computer readable medium for generating interaction diagrams for a process is disclosed. The non-transitory computer readable medium includes instructions stored thereon that when processed by a processor causes a system to perform one or more acts. First act is receiving a first oxygen saturation level and a second oxygen saturation level from at least two pulse oximetry sensors placed on the subject, wherein the first oxygen saturation level is an oxygen saturation level in incoming blood flow via a carotid artery of the subject and the second oxygen saturation level is an oxygen saturation level in outgoing blood flow via a jugular vein of the subject. Next, act is to determine cerebral extraction of oxygen using the first and second oxygen saturation levels by determining a difference between the first and second oxygen saturation levels. Next, act is to compare the cerebral extraction of oxygen with a threshold level and identify a mental fatigue level of the subject based on outcome of the comparison. The mental fatigue level indicates the health condition of the subject.

The foregoing summary is illustrative only and is not intended to be in any way limiting. In addition to the illustrative aspects, embodiments, and features described above, further aspects, embodiments, and features will become apparent by reference to the drawings and the following detailed description.

**BRIEF DESCRIPTION OF THE ACCOMPANYING DRAWINGS**

The accompanying drawings, which are incorporated in and constitute a part of this disclosure, illustrate exemplary embodiments and, together with the description, serve to explain the disclosed principles. In the figures, the left-most digit(s) of a reference number identifies the figure in which the reference number first appears. The same numbers are used throughout the figures to reference like features and components. Some embodiments of system and/or methods in accordance with embodiments of the present subject matter are now described, by way of example only, and with reference to the accompanying figures, in which:

**Fig. 1A** is a schematic illustration of the neck region of a human body;

**Fig. 1B** illustrates a block diagram of an exemplary computing unit to monitor health condition of a subject in accordance with some embodiments of the present disclosure;

**Fig. 2A** illustrates a block diagram of an exemplary computing unit to monitor health condition of a subject and display the mental fatigue level on an associated display in accordance with some embodiments of the present disclosure;

**Fig. 2B** illustrates a block diagram of an exemplary computing unit to monitor health condition of a subject and an associated display unit for displaying mental fatigue level in accordance with some embodiments of the present disclosure;

**Fig. 3A** illustrates an environment in which a computing unit receives physiological signals associated with a subject in accordance with some embodiments of the present disclosure;

**Fig. 3B** illustrates an environment in which a computing unit receives physiological signals from a plurality of subjects in accordance with some embodiments of the present disclosure;

**Fig. 4A** and **4B** illustrate exemplary fatigue charts representing of a mental fatigue level of a subject in accordance with some embodiments of the present disclosure;

**Fig. 5** illustrates an exemplary environment in which health condition of a human is monitored using an exemplary computing unit in accordance with an example embodiment of the present disclosure;

**Fig. 6** shows a flowchart illustrating a method of determining health condition of a subject using a computing device in accordance with some embodiments of the present disclosure; and

**Figure 7** illustrates a block diagram of an exemplary computer system for implementing embodiments consistent with the present disclosure.

It should be appreciated by those skilled in the art that any block diagrams herein represent conceptual views of illustrative systems embodying the principles of the present subject matter. Similarly, it will be appreciated that any flow charts, flow diagrams, state transition diagrams, pseudo code, and the like represent various processes which may be substantially represented in computer readable medium and executed by a computer or processor, whether or not such computer or processor is explicitly shown.

## DETAILED DESCRIPTION

In the present document, the word "exemplary" is used herein to mean "serving as an example, instance, or illustration." Any embodiment or implementation of the present subject matter described herein as "exemplary" is not necessarily to be construed as preferred or advantageous over other embodiments.

The terms "comprises", "comprising", or any other variations thereof, are intended to cover a non-exclusive inclusion, such that a setup, device or method that comprises a list of components or steps does not include only those components or steps but may include other components or steps not expressly listed or inherent to such setup or device or method. In other words, one or more elements in a system or apparatus proceeded by "comprises... a" does not, without more constraints, preclude the existence of other elements or additional elements in the system or apparatus.

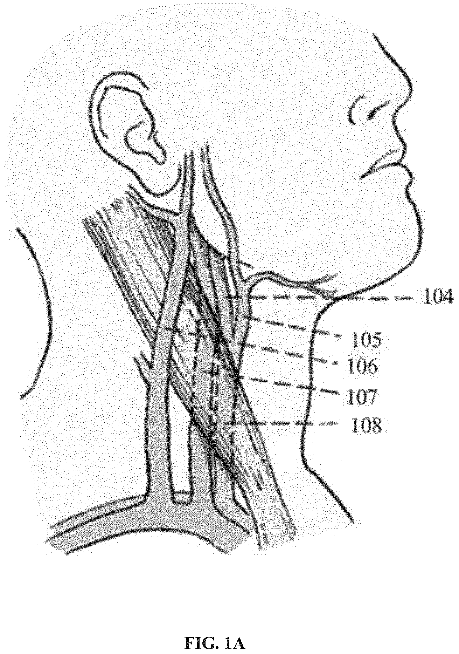

Embodiments of the present disclosure are related to a method and a system for determining health condition of a subject. In particular, the present disclosure relates to determining mental fatigue level of a subject. The subject may be a human being or an animal. In the present disclosure, a human being has been used as an exemplary subject to explain the working of the present disclosure. Further, some of the basic vascular anatomy associated with the cardiovascular system of a human being is illustrated in **Fig. 1A. Fig. 1A** is a schematic illustration of the neck region of a human body showing some of the major arteries and veins of the cardiovascular system. In an embodiment, common carotid arteries **108** are arteries that supply the head and neck including brain with oxygenated blood. The common carotid artery **108** divides in the neck to form the internal carotid artery **104** and external carotid artery **105.** The jugular veins are veins that bring deoxygenated blood from the head back to the heart. Each side of the neck has two jugular veins, external jugular vein **106** and internal jugular vein **107.** The external jugular vein **106** carries blood from the face, neck, and scalp and has two branches, posterior and anterior. The internal jugular vein **107** receives blood from the brain, the deeper tissues of the neck and the interior of the skull.

The method disclosed in the present disclosure measures the oxygen levels consumed by the brain while the subject is continuously performing an activity. Here, the activity indicates a task performed by the subject continuously which involves taking appropriate decisions, for example driving, technician in an assembly line, stock trader, etc. In an embodiment, the method comprises sensing oxygen saturation level in a noninvasive manner by using one or more sensors on the body of the subject. Then, the sensed oxygen saturation level is collected using a data aggregator. The data aggregator transmits the oxygen saturation levels to a computing unit. The computing unit computes Cerebral Extraction of Oxygen (CEO2) indicating the oxygen levels of the blood flowing through the brain. The cerebral extraction of oxygen is then compared with a threshold level to identify a mental fatigue level of the subject. In an embodiment, the threshold levels may be personalized for each subject using the historical fatigue trends. Finally, the mental fatigue level of the subject is displayed on a display unit. In an embodiment, a measurement error may be detected while determining the mental fatigue level.

The term "health condition" includes, but is not limited to, the fatigue of the subject. The term "fatigue" in ordinary describes a very common phenomenon. For purpose of this disclosure "fatigue" comprises and may be defined as: - awareness of a decreased capacity for physical and/or mental activity due to an imbalance in the availability, utilization, and/or restoration of resources needed to perform activity - a state of weariness related to reduced motivation a transitional state between wakefulness and sleep physical state of disturbed homeostasis due to work or stress, which manifest in loss in efficiency and a general disinclination to work - a feeling of weariness and inability to mobilize energy. Onset of fatigue is associated with increased anxiety, decreased short term memory, slowed reaction time, decreased work efficiency, reduced motivational drive, decreased vigilance, increased variability in work performance, increased errors and omissions which increase when time pressure, diminishing of information processing and sustained attention. The term "fatigue" used in the disclosure may be understood to comprise also any term mentioned below so for purposes of this disclosure. Following terms characterizing fatigue may be considered as synonyms. They are: exhaustion, lack of motivation, tiredness, boredom, sleepiness, feeling tired and listless, apathy, indifference, inertia, lethargy, stolidity, vacancy, drowsiness, depletion, feeling weary, feeling tired, strained or sleepy, being tired, being sleepy, being drained, being worn out, being spent, overworked. Also, fatigue can be suitably understood as opposite to following terms: vigilance, alertness, watchfulness, and wakefulness. Any of these terms as for example lack of vigilance, lack of alertness, can be also suitably treated as replacement of word fatigue in accordance with this disclosure.

In the following detailed description of the embodiments of the disclosure, reference is made to the accompanying drawings that form a part hereof, and in which are shown by way of illustration specific embodiments in which the disclosure may be practiced. These embodiments are described in sufficient detail to enable those skilled in the art to practice the disclosure, and it is to be understood that other embodiments may be utilized and that changes may be made without departing from the scope of the present disclosure. The following description is, therefore, not to be taken in a limiting sense.

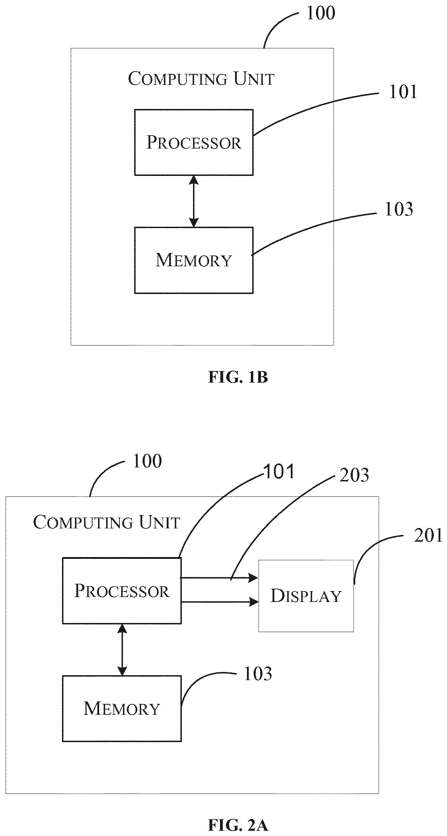

**Fig. 1B** illustrates an exemplary computing unit **100** adopted for determining health condition of a subject in accordance with some embodiments of the present disclosure. The computing unit **100** may include at least one central processing unit ("CPU" or "processor") **101** and a memory **103** storing instructions executable by the at least one processor **101**. The instructions configure the processor **101** to receive oxygen saturation levels from a plurality of sensors placed on the subject. The subject may be one of human being and animal.

The processor **101** may comprise at least one data processor for executing program components for executing user- or system-generated requests. A user may include a person, a person using a device such as those included in this disclosure, or such a device itself.

The sensors measure oxygen saturation levels of the subject. In an example, the sensors are configured on a patch which is positioned on the neck of the subject. In an embodiment, a first sensor captures a first oxygen saturation level (SaO2) in the incoming blood flow to brain via carotid artery of the subject. A second sensor captures a second oxygen saturation level (SjvO2) in an outgoing blood flow from brain via jugular vein of the subject. The first and second oxygen saturation levels are measured simultaneously at a predefined interval of time to determine the oxygen levels consumed by the brain. Initially, the processor **101** determines cerebral extraction of oxygen based on the first and second oxygen saturation levels from the sensors. The cerebral extraction of oxygen is determined by calculating a difference between the first and second oxygen saturation levels as shown below.

$$CEO2_i = (((SaO_2)_i - (SjvO_2)_i) / (SaO_2)_i) * 100 \qquad \text{---------------------------------- (1)}$$

where '$i$' indicate $i^{th}$ sample in the time sequence.

The trend of CEO2 indicates the oxygen consumption of brain and mental stress over a period of time.

Thereafter, the processor **101** generates a mental fatigue level by comparing the cerebral extraction of oxygen with a predefined threshold level. The mental fatigue level indicates the health condition of the subject. If the values of CEO2 are above a threshold level for a predefined interval of time, then the mental fatigue of the subject is detected.

In some embodiments of the present disclosure, the computing unit **100** may comprise an alert system (not shown) for generating an alarm. The alarm may be generated if the mental fatigue level is substantially close to or greater than the predefined threshold level.

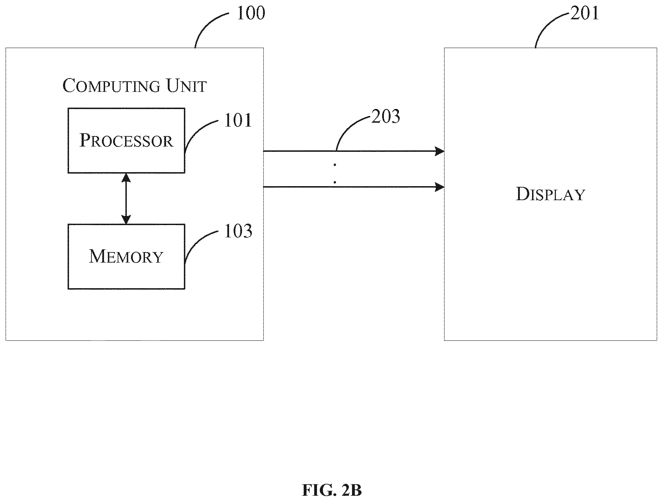

**Figs. 2A** and **2B** illustrates a computing unit **100** to monitor health condition of a subject and display the mental fatigue level on an associated display unit, in accordance with some embodiments of the present disclosure. In some embodiments, the computing unit **100** may comprise at least one processor **101,** a memory **103** storing instructions executable by the at least one processor **101** and a display unit **201** for displaying mental fatigue information **203** of at least one subject, as shown in **Fig. 2A.** In some embodiments, the computing unit may comprise at least one processor **101,** a memory **103** storing instructions executable by the at least one processor **101** and an associated external display unit **201** for displaying mental fatigue information **203** of at least one subject, as shown in **Fig. 2B.** The computing unit **100** transmits the mental fatigue information **203** such as, but not limited to, mental fatigue level, number of subjects for which fatigue score is generated, to the display unit **201.** The display unit **201** displays the fatigue information, as received from the computing unit **100.**

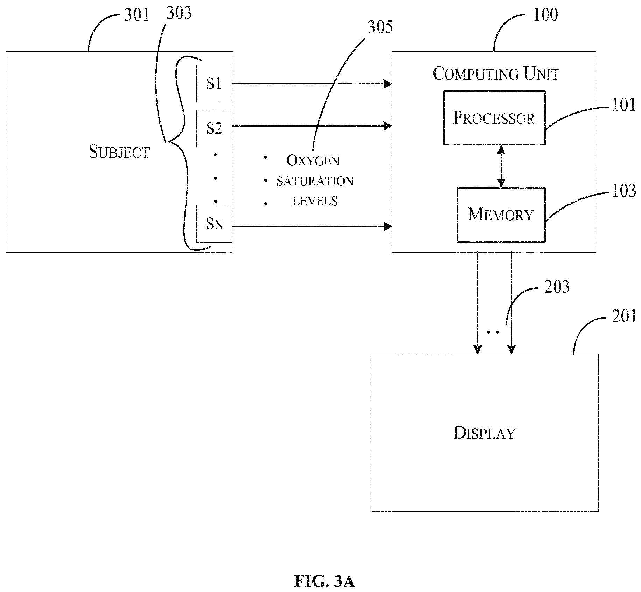

**Fig. 3A** illustrates an environment in which a computing unit **100** receives signals associated with a subject in accordance with some embodiments of the present disclosure. The computing unit **100** may be configured to receive oxygen saturation levels **305** from a plurality of sensors (S1, S2... Sn) **303** placed on the subject **301.** The subject **301** may be one of a human being and an animal. The oxygen saturation levels may be received from plurality of sensors such as pulse oximetry sensors. A person skilled in the art would understand that any other sensor which provides oxygen saturation levels of blood can be used with the method of the present disclosure.

In an exemplary embodiment, the sensors **303** may be placed in the form of adhesive patches on the body of a subject **301** such as, but not limited to a worker or an employee. For example, a pulse oximetry sensor may be placed on the neck region of the worker. In particular, a first sensor is to be placed on the carotid artery and the second sensor is to be places on jugular vein.

The computing unit **100** may receive the signals **305** from the plurality of sensors (S1, S2... Sn) **303,** using either wired or wireless means. In one exemplary embodiment, the computing unit **100** may receive the physiological signals **305** using wireless radio technology such as, but not limited to, WiFi, Bluetooth and Zigbee. Further, the computing unit **100** may include a data aggregator for acquiring the physiological signals **305** from plurality of sensors **303.** The data aggregator may be placed in the vicinity of the subject for data aggregation. Upon acquiring the physiological signals **305** by the data aggregator, the signals are stored in the memory **103** for further processing.

The processor **101** may estimate a mental fatigue level from the oxygen saturation levels **305** using sensor specific methodologies. The signals **305** are received by the computing unit **100** from the plurality of sensors **303,** wherein each sensor signals are converted into digital data and queued by the data aggregator. The queued data is processed using sensor specific method. The estimated fatigue values are stored in the memory of the computing unit **101** for further processing.

The processor **101** computes the cerebral extraction of oxygen for each sample of oxygen saturation levels received for duration of predefined amount of time. Then, the processor **101** computes a median value of the sets of windowed data of cerebral extraction of oxygen. This is done to avoid outliers which are caused due to sampling or measurement errors.

Then processor **101** then analyzes the trend of cerebral extraction of oxygen to determine the health condition of the subject. The fatigue is a slow varying characteristic, therefore the values of CEO2 for a predefined amount of time are taken into consideration. For example, the CEO2 values of recent 5 minutes can be considered for analysis. In an embodiment, the CEO2 values are analyzed by comparing the CEO2 values with the threshold level.

In an embodiment, single threshold level may be used with the method of present disclosure. If the CEO2 value of the subject is substantially near or equal to the threshold level, then an alarm is generated by the computing unit **100** to notify the subject or any other system or person regarding the health condition of the subject.

The threshold levels may also be classified at least one of low threshold level, medium threshold level and high threshold level. As an example, the CEO2 of the subject can be compared with at least one of these levels to monitor health condition of the subject. In an embodiment, if the CEO2 value of the subject is substantially near or equal to the low threshold level, then the subject is considered to have low mental fatigue. In such a case, the subject can perform tasks without any difficulty/risk. If the CEO2 value of the subject is substantially near or equal to the medium threshold level, then the subject is considered to have medium mental fatigue. In such a case, the subject can perform tasks, however fatigue level of the subject needs to be monitored continuously. In the alternative, if the CEO2 value is substantially near or equal to the high threshold level, then the subject needs to take rest and should not be allowed to perform tasks that require concentration.

The threshold level may be created based on a population database. The population database comprises values of cerebral extraction of oxygen for a large population with various age groups. These values are used to create the threshold levels for different age groups. The below table, **Table 1** shows exemplary values of threshold levels for different age groups.

Table 1

| Age (in years) | Low threshold level | Medium threshold level | High threshold level |
|:---:|:---:|:---:|:---:|
| <20 | 80 | 85 | 90 |
| 20-30 | 75 | 80 | 85 |
| 30-40 | 70 | 80 | 85 |
| 40-50 | 65 | 75 | 80 |
| 50-70 | 60 | 70 | 75 |
| >70 | 50 | 60 | 70 |

In an embodiment, the threshold levels may be personalized for each subject, as different subjects have different health conditions and the fatigue level may not be same for all the subjects. The threshold levels may be personalized for a subject by considering CEO2 levels of the subject during resting, normal activity and restlessness scenarios over a period of time.

As an example of personalization of the threshold values, the PERSONAL_OFFSET of a subject can be calculated using the CEO2 value during rest. For different subjects of same age group, the range of CEO2 may vary depending on the variations in the blood flow to brain. This can be normalized by using the PERSONAL_OFFSET. The final CEO2 value for trend analysis is calculated as shown below:

$$PERSONAL\_OFFSET = Individual\_Rest\_CEO2 - Population\_Rest\_CEO2 \quad \text{------- (2)}$$

$$Final\_CEO2\_Val = Measured\_CEO2 - (Wt1 * PERSONAL\_OFFSET) \quad \text{------------- (3)}$$

where,

Individual_Rest_CEO2 is CEO2 measured during resting of the individual,
Population_Rest_CEO2 is CEO2 of the population database of similar age group during rest,
Measured_CEO2 is CEO2 of the individual measured using carotid artery and jugular vein blood saturation values, and
Wt1 is weightage given to personal offset value which ranges from 0 to 1. In an embodiment, the weightage is assigned based on information in the population database.

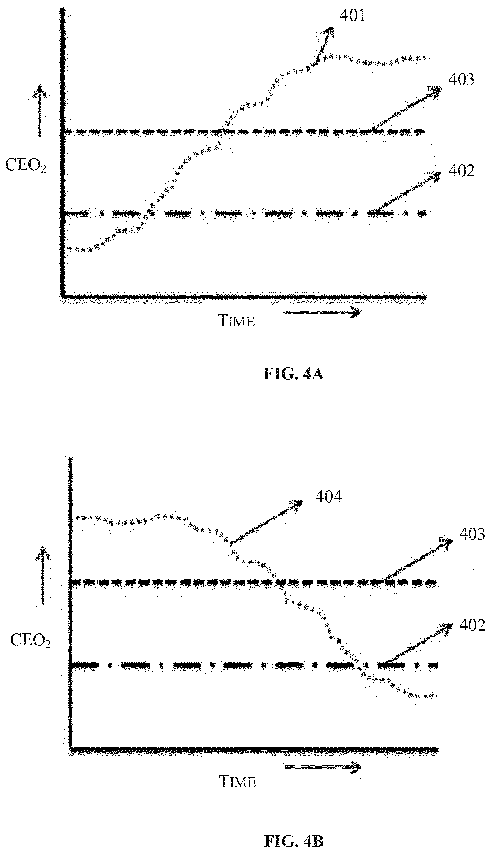

Upon comparison of the CEO2 values with the threshold levels, the processor **101** identifies a mental fatigue level of the subject. The mental fatigue level indicates the health condition of the subject. After determining the health condition of the subject, the processor **101** may display the mental fatigue level on the associated display unit **203**. **Fig. 4A** and **4B** illustrate exemplary fatigue charts representing mental fatigue level of a subject in accordance with some embodiments of the present disclosure. **Fig. 4A** illustrates an increasing fatigue condition **401** for a subject, where the mental fatigue level of the subject is increasing with time. **Fig. 4B** illustrates decreasing fatigue condition **404** for a subject, where the mental fatigue level of the subject is decreasing with time. In both the fatigue charts, only two threshold levels, i.e. medium threshold level **402** and high threshold level **403** have been considered for determining health condition of the patient. Using the trend in the fatigue chart, the supervisor of the subject may change or reschedule the worker job assignments for the subject.

In some embodiments of the present disclosure, the computing unit **100** may generate an alarm to indicate of the mental CEO2 level is above nearing, equal or above the predefined threshold level. The alarm may be displayed using image, video, text on the display **201.** Alternatively or in addition, the alarm may be provided to the subject or other system using audio.

In an embodiment, the computing unit **100** detects a measurement error while determining the mental fatigue level. The status of the mental fatigue level at each time interval may be aggregated and analyzed to avoid any spurious or false values. Also, the analyzing of the status would help in generating realistic mental fatigue status. As an example, statistical models such as Bayes theorem may be used for detecting the measurement error while determining the mental fatigue level.

An exemplary scenario of aggregated mental fatigue status over a period of time is shown below. Here each value indicates the status of mental fatigue during a time window of 'X' minutes, say 5 minutes.

| N | N | N | M | M | M | H | H | H | H |
|---|---|---|---|---|---|---|---|---|---|

where,

N - Normal
M - Medium Fatigue
S - High Fatigue

For example, the status continuously shows medium fatigue for 5 minutes and in between there is one high fatigue status. In such a case, the high fatigue status may be due to measurement error. This may happen if the sensor is defective, the sensor is not placed properly on the subject, transmission error etc. This information may be passed as input to the post processing to evaluate real fatigue situation.

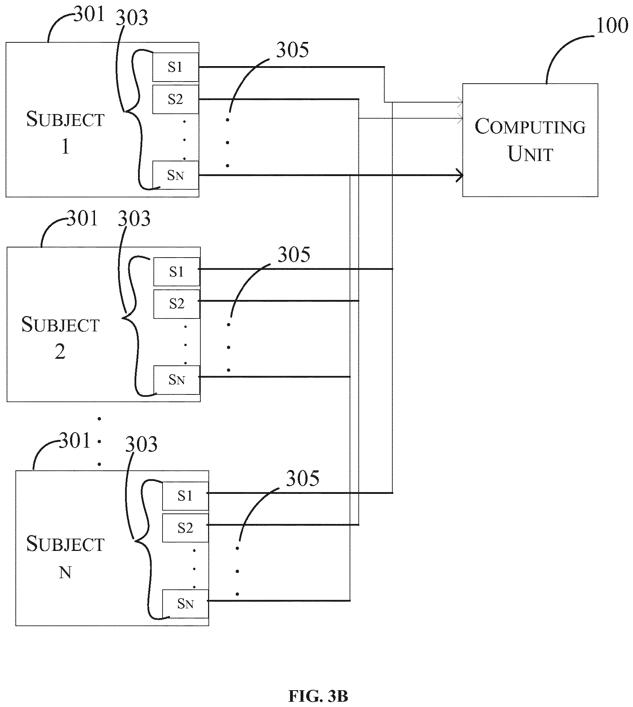

**Fig. 3B** illustrates an environment in which a computing unit **100** receives oxygen saturation levels **305** from a plurality of subjects (subject 1, subject 2... subject n) **301** in accordance with some embodiments of the present disclosure. The computing unit **100** includes at least one processor **101** and a memory **103** storing instructions executable by the at least one processor **101**. Initially, the processor **101** determines cerebral extraction of oxygen using the oxygen saturation levels. Thereafter, the processor **101** identifies the mental fatigue level of the subject by comparing the cerebral extraction of oxygen with a threshold level. The mental fatigue level indicates the health condition of the subject.

The computing unit may further comprise an alert system for generating an alarm. In some embodiment, the fatigue information of each subject or worker may be displayed on a display **201** by the computing unit **100**.

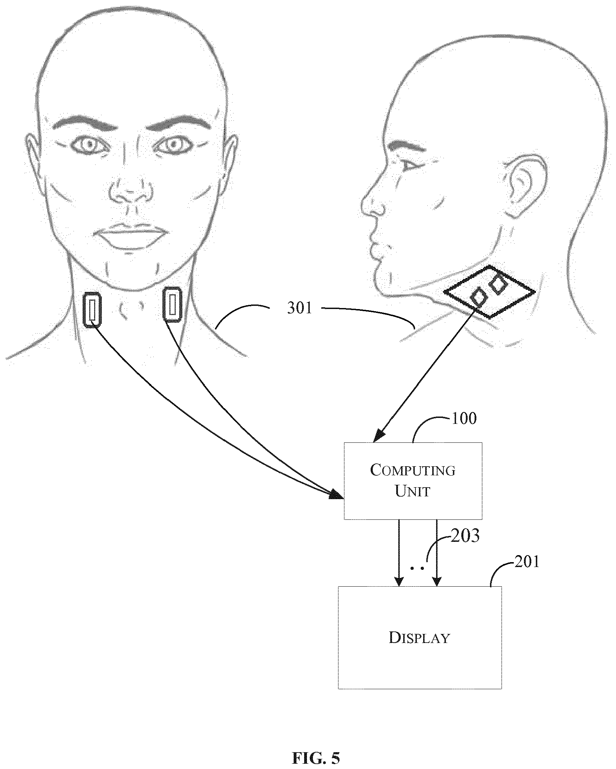

**Fig. 5** illustrates an exemplary computing unit **100** to monitor health condition of a human **301** along with an associated display **201** in accordance with an example embodiment of the present disclosure. The computing unit **100** may be configured to receive oxygen saturation levels **305** from a plurality of sensors (S1, S2... Sn) **303** placed on the human **301**. Particularly, in the illustrated figure, two sensors are placed on the neck of the subject using a patch. The sensors may be placed on sides of the neck. Also, the two sensors may be put on a single patch and placed on one side of the neck. In some exemplary embodiments, the oxygen saturation levels may be transmitted by a transmitter **501** placed along with the plurality of sensors, on the body of a human **301.**

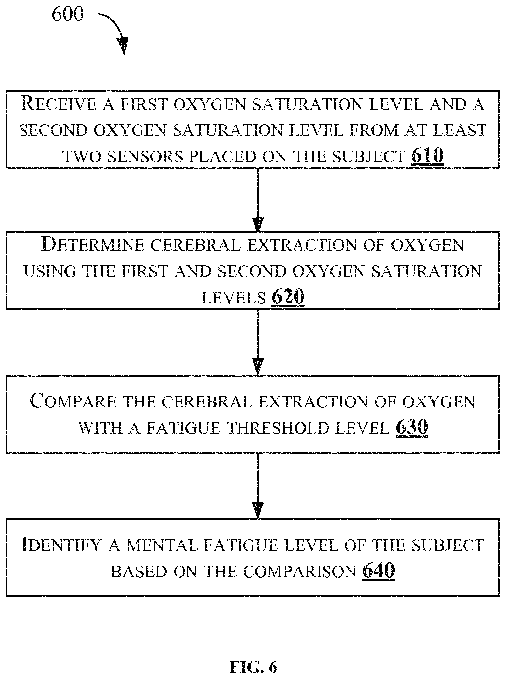

**Fig. 6** shows a flowchart illustrating a method for determining health condition of a subject using a computing unit in accordance with some embodiments of the present disclosure.

As illustrated in **Figure 6,** the method **600** comprises one or more blocks for determining health condition of a subject by the computing unit **100**. The method **600** may be described in the general context of computer executable instructions. Generally, computer executable instructions can include routines, programs, objects, components, data structures, procedures, modules, and functions, which perform particular functions or implement particular abstract data types.

The method **600** can be implemented in any suitable hardware, software, firmware, or combination thereof.

At **block 610,** receive a first oxygen saturation level and a second oxygen saturation level from at least two sensors placed on the subject. The at least two sensors of sensors are placed on neck of the subject. The subject may be one of human and animal. In an embodiment, the first oxygen saturation level is oxygen saturation level in the incoming blood flow via carotid artery of the subject. The second oxygen saturation level is oxygen saturation level in an outgoing blood flow via jugular vein of the subject. The first oxygen saturation level and the second oxygen saturation level are received at a predefined time interval from the at least two sensors.

At **block 620,** determine cerebral extraction of oxygen using the first and second oxygen saturation levels. The cerebral extraction of oxygen is determined by a difference between the first and the second oxygen saturation levels.

At **block 630,** compare the cerebral extraction of oxygen with a threshold level. The threshold level may be classified as at least one of low threshold level, medium threshold level and high threshold level.

At **block 640,** identify a mental fatigue level of the subject based on outcome of the comparison. The comparison of the cerebral extraction of oxygen with the threshold levels provides a mental fatigue level of the subject. The mental fatigue level indicates the health condition of the subject. In an embodiment, if the mental fatigue level is greater than at least one of medium threshold level and high threshold level, the computing unit generates an alarm signal. The alarm signal may be provided to display unit in form of audio/image/video.

Computer System

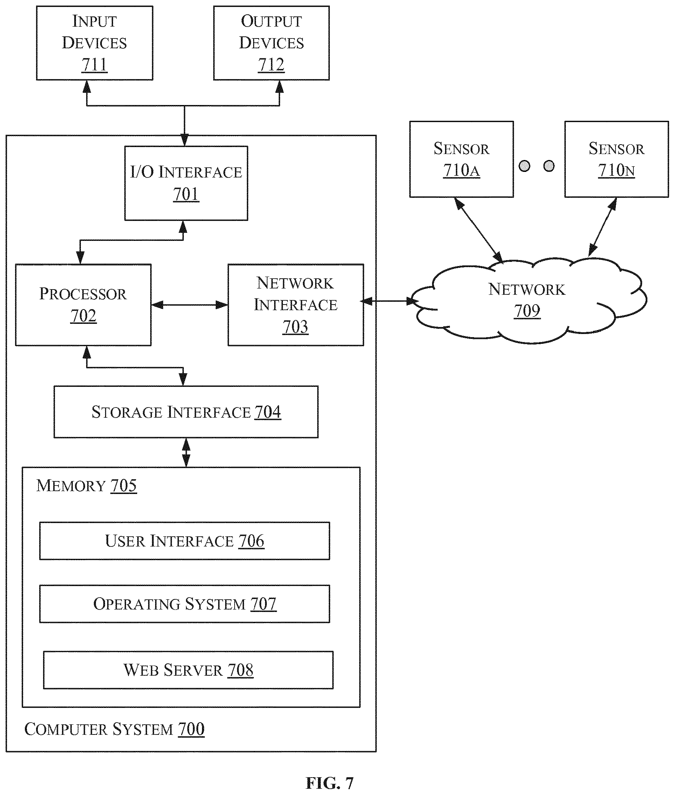

**Figure 7** illustrates a block diagram of an exemplary computer system 700 for implementing embodiments consistent with the present disclosure. In an embodiment, the computer system **700** is used to implement the computing unit **100**. The computer system **700** monitors the health condition of a subject. The computer system **700** may comprise a central processing unit ("CPU" or "processor") **702**. The processor **702** may comprise at least one data processor for executing program components for executing user- or system-generated business processes. A user may include a person, a person using a device such as those included in this disclosure, or such a device itself. The processor **702** may include specialized processing units such as integrated system (bus) controllers, memory management control units, floating point units, graphics processing units, digital signal processing units, etc.

The processor **702** may be disposed in communication with one or more input/output (I/O) devices **(711** and **712)** via I/O interface **701.** The I/O interface **701** may employ communication protocols/methods such as, without limitation, audio, analog, digital, monoaural, RCA, stereo, IEEE-1394, serial bus, universal serial bus (USB), infrared, PS/2, BNC, coaxial, component, composite, digital visual interface (DVI), high-definition multimedia interface (HDMI), RF antennas, S-Video, VGA, IEEE 802.n /b/g/n/x, Bluetooth, cellular (e.g., code-division multiple access (CDMA), high-speed packet access (HSPA+), global system for mobile communications (GSM), long-term evolution (LTE), WiMax, or the like), etc.

Using the I/O interface **701,** the computer system **700** may communicate with one or more I/O devices **(711** and **712).** For example, the input device **711** may be an antenna, keyboard, mouse, joystick, (infrared) remote control, camera, card reader, fax machine, dongle, biometric reader, microphone, touch screen, touchpad, trackball, stylus, scanner, storage device, transceiver, video device/source, etc. The output device **712** may be a printer, fax machine, video display (e.g., cathode ray tube (CRT), liquid crystal display (LCD), light-emitting diode (LED), plasma, Plasma display panel (PDP), Organic light-emitting diode display (OLED) or the like), audio speaker, etc.

In some embodiments, the processor **702** may be disposed in communication with a communication network **709** via a network interface **703**. The network interface **703** may communicate with the communication network **709.** The network interface **703** may employ connection protocols including, without limitation, direct connect, Ethernet (e.g., twisted pair 10/100/1000 Base T), transmission control protocol/internet protocol (TCP/IP), token ring, IEEE 802.11a/b/g/n/x, etc. The communication network **709** may include, without limitation, a direct interconnection, local area network (LAN), wide area network (WAN), wireless network (e.g., using Wireless Application Protocol), the Internet, etc. Using the network interface **703** and the communication network **709,** the computer system **700** may communicate with data aggregator or sensors **710.**

In some embodiments, the processor **702** may be disposed in communication with a memory **705** (e.g., RAM, ROM, etc. not shown in **figure 7)** via a storage interface **704.** The storage interface **704** may connect to memory **705** including, without limitation, memory drives, removable disc drives, etc., employing connection protocols such as serial advanced technology attachment (SATA), Integrated Drive Electronics (IDE), IEEE-1394, Universal Serial Bus (USB), fiber channel, Small Computer Systems Interface (SCSI), etc. The memory drives may further include a drum, magnetic disc drive, magneto-optical drive, optical drive, Redundant Array of Independent Discs (RAID), solid-state memory devices, solid-state drives, etc.

The memory **705** may store a collection of program or database components, including, without limitation, user interface application **706,** an operating system **707,** web server **708** etc. In some embodiments, computer system **700** may store user/application data **706,** such as the data, variables, records, etc. as described in this disclosure. Such databases may be implemented as fault-tolerant, relational, scalable, secure databases such as Oracle or Sybase.

The operating system **707** may facilitate resource management and operation of the computer system **700.** Examples of operating systems include, without limitation, Apple Macintosh OS X, Unix, Unix-like system distributions (e.g., Berkeley Software Distribution (BSD), FreeBSD, NetBSD, OpenBSD, etc.), Linux distributions (e.g., Red Hat, Ubuntu, Kubuntu, etc.), IBM OS/2, Microsoft Windows (XP, Vista/7/8, etc.), Apple iOS, Google Android, Blackberry OS, or the like. User interface **717** may facilitate display, execution, interaction, manipulation, or operation of program components through textual or graphical facilities. For example, user interfaces may provide computer interaction interface elements on a display system operatively connected to the computer system **700,** such as cursors, icons, check boxes, menus, scrollers, windows, widgets, etc. Graphical user interfaces (GUIs) may be employed, including, without limitation, Apple Macintosh operating systems' Aqua, IBM OS/2, Microsoft Windows (e.g., Aero, Metro, etc.), Unix X-Windows, web interface libraries (e.g., ActiveX, Java, Javascript, AJAX, HTML, Adobe Flash, etc.), or the like.

In some embodiments, the computer system **700** may implement a web browser **708** stored program component. The web browser may be a hypertext viewing application, such as Microsoft Internet Explorer, Google Chrome, Mozilla Firefox, Apple Safari, etc. Secure web browsing may be provided using HTTPS (secure hypertext transport protocol), secure sockets layer (SSL), Transport Layer Security (TLS), etc. Web browsers may utilize facilities such as AJAX, DHTML, Adobe Flash, JavaScript, Java, application programming interfaces (APIs), etc. In some embodiments, the computer system **700** may implement a mail server **719** stored program component. The mail server may be an Internet mail server such as Microsoft Exchange, or the like. The mail server may utilize facilities such as ASP, ActiveX, ANSI C++/C#, Microsoft .NET, CGI scripts, Java, JavaScript, PERL, PHP, Python, WebObjects, etc. The mail server may utilize communication protocols such as Internet Message Access Protocol (IMAP), Messaging Application Programming Interface (MAPI), Microsoft Exchange, Post Office Protocol (POP), Simple Mail Transfer Protocol (SMTP), or the like. In some embodiments, the computer system **700** may implement a mail client stored program component. The mail client may be a mail viewing application, such as Apple Mail, Microsoft Entourage, Microsoft Outlook, Mozilla Thunderbird, etc.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include Random Access Memory (RAM), Read-Only Memory (ROM), volatile memory, nonvolatile memory, hard drives, CD ROMs, DVDs, flash drives, disks, and any other known physical storage media.

Advantages of the embodiment of the present disclosure are illustrated herein.

Embodiment of the present disclosure provides noninvasive measurement of mental fatigue of a subject.

The method of present disclosure measuring oxygen saturation levels of carotid artery and jugular vein provides more reliable readings of mental fatigue as fatigue is state of brain's alertness.

In an embodiment of the present disclosure, any measurement errors while determining the mental fatigue level can be detected.

The described operations may be implemented as a method, system or article of manufacture using standard programming and/or engineering techniques to produce software, firmware, hardware, or any combination thereof. The described operations may be implemented as code maintained in a "non-transitory computer readable medium", where a processor may read and execute the code from the computer readable medium. The processor is at least one of a microprocessor and a processor capable of processing and executing the queries. A non-transitory computer readable medium may comprise media such as magnetic storage medium (e.g., hard disk drives, floppy disks, tape, etc.), optical storage (CD-ROMs, DVDs, optical disks, etc.), volatile and nonvolatile memory devices (e.g., EEPROMs, ROMs, PROMs, RAMs, DRAMs, SRAMs, Flash Memory, firmware, programmable logic, etc.), etc. Further, non-transitory computer-readable media comprise all computer-readable media except for a transitory. The code implementing the described operations may further be implemented in hardware logic (e.g., an integrated circuit chip, Programmable Gate Array (PGA), Application Specific Integrated Circuit (ASIC), etc.).

Still further, the code implementing the described operations may be implemented in "transmission signals", where transmission signals may propagate through space or through a transmission media, such as an optical fiber, copper wire, etc. The transmission signals in which the code or logic is encoded may further comprise a wireless signal, satellite transmission, radio waves, infrared signals, Bluetooth, etc. The transmission signals in which the code or logic is encoded is capable of being transmitted by a transmitting station and received by a receiving station, where the code or logic encoded in the transmission signal may be decoded and stored in hardware or a non-transitory computer readable medium at the receiving and transmitting stations or devices. An "article of manufacture" comprises non-transitory computer readable medium, hardware logic, and/or transmission signals in which code may be implemented. A device in which the code implementing the described embodiments of operations is encoded may comprise a computer readable medium or hardware logic. Of course, those skilled in the art will recognize that many modifications may be made to this configuration without departing from the scope of the invention, and that the article of manufacture may comprise suitable information bearing medium known in the art.

The terms "an embodiment", "embodiment", "embodiments", "the embodiment", "the embodiments", "one or more embodiments", "some embodiments", and "one embodiment" mean "one or more (but not all) embodiments of the invention(s)" unless expressly specified otherwise.

The terms "including", "comprising", "having" and variations thereof mean "including but not limited to", unless expressly specified otherwise.

The enumerated listing of items does not imply that any or all of the items are mutually exclusive, unless expressly specified otherwise.

The terms "a", "an" and "the" mean "one or more", unless expressly specified otherwise.

A description of an embodiment with several components in communication with each other does not imply that all such components are required. On the contrary a variety of optional components are described to illustrate the wide variety of possible embodiments of the invention.

When a single device or article is described herein, it will be readily apparent that more than one device/article (whether or not they cooperate) may be used in place of a single device/article. Similarly, where more than one device or article is described herein (whether or not they cooperate), it will be readily apparent that a single device/article may be used in place of the more than one device or article or a different number of devices/articles may be used instead of the shown number of devices or programs. The functionality and/or the features of a device may be alternatively embodied by one or more other devices which are not explicitly described as having such functionality/features. Thus, other embodiments of the invention need not include the device itself.

The illustrated operations of **Figure 6** show certain events occurring in a certain order. In alternative embodiments, certain operations may be performed in a different order, modified or removed. Moreover, steps may be added to the above described logic and still conform to the described embodiments. Further, operations described herein may occur sequentially or certain operations may be processed in parallel. Yet further, operations may be performed by a single processing unit or by distributed processing units.

Finally, the language used in the specification has been principally selected for readability and instructional purposes, and it may not have been selected to delineate or circumscribe the inventive subject matter. It is therefore intended that the scope of the invention be limited not by this detailed description, but rather by any claims that issue on an application based here on. Accordingly, the disclosure of the embodiments of the invention is intended to be illustrative, but not limiting, of the scope of the invention, which is set forth in the following claims.

While various aspects and embodiments have been disclosed herein, other aspects and embodiments will be apparent to those skilled in the art. The various aspects and embodiments disclosed herein are for purposes of illustration and are not intended to be limiting, with the scope being indicated by the following claims

**Claims**

1.  A method for determining a health condition of a subject (301), the method comprising:

    receiving (610), by a computing unit (100), a first oxygen saturation level (305) and a second oxygen saturation level (305) from at least two pulse oximetry sensors (303) placed on the subject, wherein the first oxygen saturation level is an oxygen saturation level in incoming blood flow via a carotid artery (104, 105) of the subject and the second oxygen saturation level is an oxygen saturation level in outgoing blood flow via a jugular vein (106, 107) of the subject;
    determining (620), by the computing unit, cerebral extraction of oxygen by determining a difference between the first and second oxygen saturation levels;
    comparing (630), by the computing unit, the cerebral extraction of oxygen with a threshold level; and identifying (640), by the computing unit, a mental fatigue level of the subject based on the comparison, wherein the mental fatigue level indicates the health condition of the subject.

2.  The method as claimed in claim 1, wherein the first oxygen saturation level and the second oxygen saturation level are received at a predefined time interval.

3.  The method as claimed in claim 1 or 2, further comprising displaying at least one of the mental fatigue level and the threshold level on a display unit (201) associated with the computing unit.

4.  The method as claimed in any of the preceding claims, wherein comparing the cerebral extraction of oxygen with the threshold level comprises comparing the cerebral extraction of oxygen with at least one of a low threshold level, a medium threshold level and a high threshold level.

5.  The method as claimed in claim 4, further comprising generating an alarm if the mental fatigue level is greater than at least one of the medium threshold level (402) and the high threshold level (403).

6.  The method as claimed in any of the preceding claims, wherein the threshold level is personalized based on at least one of age of the subject, historical data of cerebral extraction of oxygen of the subject.

7.  A computing unit (100) for determining a health condition of a subject (301), comprising:

    a processor (101); and
    a memory (103) communicatively coupled to the processor and storing processor-executable instructions, which, on execution, cause the processor to perform the method of any of the preceding claims.

8.  A computer readable medium including instructions stored thereon that when processed by at least one processor (101) cause the processor to perform the method of any of claims 1 to 6.


**Patentansprüche**

1.  Verfahren zur Bestimmung eines Gesundheitszustands einer Person (301), wobei das Verfahren Folgendes umfasst:

    Empfangen (610), durch eine Recheneinheit (100), eines ersten Sauerstoffsättigungsgrads (305) und eines zweiten Sauerstoffsättigungsgrads (305) von zumindest zwei Pulsoximetriesensoren (303), die an der Person platziert sind,
    wobei der erste Sauerstoffsättigungsgrad ein Sauerstoffsättigungsgrad in eingehendem Blutfluss über eine Halsschlagader (104, 105) der Person ist und der zweite Sauerstoffsättigungsgrad ein Sauerstoffsättigungsgrad in ausgehendem Blutfluss über eine Halsschlagader (106, 107) der Person ist;
    Bestimmen (620), durch die Recheneinheit, von zerebraler Extraktion von Sauerstoff durch Bestimmen einer Differenz zwischen dem ersten und dem zweiten Sauerstoffsättigungsgrad;
    Vergleichen (630), durch die Recheneinheit, der zerebralen Extraktion von Sauerstoff mit einem Schwellenwertgrad; und
    Identifizieren (640), durch die Recheneinheit, eines Grads an geistiger Erschöpfung der Person basierend auf dem Vergleich, wobei der Grad an geistiger Erschöpfung den Gesundheitszustand der Person angibt.

**2.** Verfahren nach Anspruch 1, wobei der erste Sauerstoffsättigungsgrad und der zweite Sauerstoffsättigungsgrad in einem vordefinierten Zeitintervall empfangen werden.

**3.** Verfahren nach Anspruch 1 oder 2, ferner umfassend Anzeigen von zumindest einem von dem Grad an geistiger Erschöpfung und dem Schwellenwertgrad auf einer Anzeigeeinheit (201), die mit der Recheneinheit verbunden ist.

**4.** Verfahren nach einem der vorhergehenden Ansprüche, wobei das Vergleichen der zerebralen Extraktion von Sauerstoff mit dem Schwellenwertgrad Vergleichen der zerebralen Extraktion von Sauerstoff mit zumindest einem von einem niedrigen Schwellenwertgrad, einem mittleren Schwellenwertgrad und einem hohen Schwellenwertgrad umfasst.

**5.** Verfahren nach Anspruch 4, ferner umfassend Erzeugen eines Alarms, wenn der Grad an geistiger Erschöpfung größer als zumindest eines von dem mittleren Schwellenwertgrad (402) und dem hohen Schwellenwertgrad (403) ist.

**6.** Verfahren nach einem der vorhergehenden Ansprüche, wobei der Schwellenwertgrad basierend auf zumindest einem von Alter der Person, historischen Daten von zerebraler Extraktion von Sauerstoff der Person personalisiert ist.

**7.** Recheneinheit (100) zur Bestimmung eines Gesundheitszustands einer Person (301), umfassend:

einen Prozessor (101); und
einen Speicher (103), der kommunikativ an den Prozessor gekoppelt ist und prozessorausführbare Anweisungen speichert, die bei Ausführung veranlassen, dass der Prozessor das Verfahren nach einem der vorhergehenden Ansprüche durchführt.

**8.** Computerlesbares Medium, das Anweisungen beinhaltet, die darauf gespeichert sind, die, wenn sie durch zumindest einen Prozessor (101) verarbeitet werden, veranlassen, dass der Prozessor das Verfahren nach einem der Ansprüche 1 bis 6 durchführt.

## Revendications

**1.** Procédé de détermination de l'état de santé d'un sujet (301), le procédé comprenant :

la réception (610) par une unité de calcul (100) d'un premier niveau de saturation en oxygène (305) et d'un second niveau de saturation en oxygène (305) d'au moins deux capteurs d'oxymétrie de pouls (303) placés sur le sujet,
le premier niveau de saturation en oxygène étant un niveau de saturation en oxygène dans le flux sanguin entrant via une artère carotide (104, 105) du sujet et le second niveau de saturation en oxygène étant un niveau de saturation en oxygène dans le flux sanguin sortant via une veine jugulaire (106, 107) du sujet ;
la détermination (620) par l'unité de calcul de l'extraction cérébrale de l'oxygène par la détermination d'une différence entre les premier et second niveaux de saturation en oxygène ;
la comparaison (630) par l'unité de calcul de l'extraction cérébrale d'oxygène avec un niveau seuil ; et
l'identification (640) par l'unité de calcul d'un niveau de fatigue mentale du sujet basé sur la comparaison, le niveau de fatigue mentale indiquant l'état de santé du sujet.

**2.** Procédé tel que revendiqué dans la revendication 1, dans lequel le premier niveau de saturation en oxygène et le second niveau de saturation en oxygène sont reçus à un intervalle de temps prédéfini.

**3.** Procédé tel que revendiqué dans la revendication 1 ou 2, comprenant en outre l'affichage d'au moins un parmi le niveau de fatigue mentale et le niveau de seuil sur une unité d'affichage (201) associée à l'unité de calcul.

**4.** Procédé tel que revendiqué dans l'une quelconque des revendications précédentes, dans lequel la comparaison de l'extraction cérébrale d'oxygène avec le seuil comprend la comparaison de l'extraction cérébrale d'oxygène avec au moins un parmi un niveau de seuil faible, un niveau de seuil moyen et un niveau de seuil élevé.

**5.** Procédé tel que revendiqué dans la revendication 4, comprenant en outre l'émission d'une alarme si le niveau de fatigue mentale est supérieur à au moins un parmi le seuil moyen (402) et le seuil élevé (403).

6. Procédé tel que revendiqué dans l'une quelconque des revendications précédentes, dans lequel le seuil est personnalisé en fonction d'au moins un parmi l'âge du sujet, les données historiques d'extraction cérébrale d'oxygène du sujet.

7. Unité de calcul (100) pour la détermination de l'état de santé d'un sujet (301), comprenant :

   un processeur (101) ; et
   une mémoire (103) couplée de façon communicative au processeur et stockant les instructions exécutables du processeur, qui, à l'exécution, obligent le processeur à exécuter le procédé de l'une des revendications précédentes.

8. Support lisible par ordinateur comprenant des instructions qui y sont stockées et qui, lorsqu'elles sont traitées par au moins un processeur (101), obligent le processeur à exécuter le procédé selon l'une quelconque des revendications 1 à 6.

104
105
106
107
108

**FIG. 1A**

**FIG. 1B**

**FIG. 2A**

**FIG. 2B**

EP 3 001 337 B1

**FIG. 3A**

**FIG. 3B**

**FIG. 4A**

**FIG. 4B**

EP 3 001 337 B1

FIG. 5

21

600

RECEIVE A FIRST OXYGEN SATURATION LEVEL AND A SECOND OXYGEN SATURATION LEVEL FROM AT LEAST TWO SENSORS PLACED ON THE SUBJECT **610**

DETERMINE CEREBRAL EXTRACTION OF OXYGEN USING THE FIRST AND SECOND OXYGEN SATURATION LEVELS **620**

COMPARE THE CEREBRAL EXTRACTION OF OXYGEN WITH A FATIGUE THRESHOLD LEVEL **630**

IDENTIFY A MENTAL FATIGUE LEVEL OF THE SUBJECT BASED ON THE COMPARISON **640**

**FIG. 6**

FIG. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20100222658 A1
- US 20060247542 A1
- US 20110199214 A1

### Non-patent literature cited in the description

- Assessment of cerebral oxygenation during prolonged simulated driving using near infrared spectroscopy: its implications for fatigue development. **LI Z. et al.** EUROPEAN JOURNAL OF APPLIED PHYSIOLOGY. SPRINGER, 04 July 2009, vol. 107
- **NYBO L. et al.** Inadequate Cerebral Oxygen Delivery and Central Fatigue during Strenuous Exercise. *EXERCISE AND SPORT SCIENCES REVIEWS, D2,* 01 July 2007, vol. 35 (3), ISSN 0091-6331
- **JAGANNATH M. et al.** Assessment of early onset of driver fatigue using multimodal fatigue measures in a static simulator. *APPLIED ERGONOMICS, D3,* 26 February 2014, vol. 45 (4), ISSN 0003-6870